(19) Europäisches Patentamt
European Patent Office
Office européen des brevets

(11) Publication number: 0 092 226 B1

(12) **EUROPEAN PATENT SPECIFICATION**

(45) Date of publication of patent specification: **19.08.87**

(51) Int. Cl.[4]: **C 07 H 15/20**, A 61 K 31/70 // A61K35/78

(21) Application number: **83103765.0**

(22) Date of filing: **19.04.83**

(54) **Extract of plants of the family of hypoxidaceae for treatment of cancer.**

(30) Priority: **19.04.82 GB 8211294**

(43) Date of publication of application: **26.10.83 Bulletin 83/43**

(45) Publication of the grant of the patent: **19.08.87 Bulletin 87/34**

(84) Designated Contracting States: **AT BE CH DE FR GB IT LI LU NL SE**

(56) References cited:
**DE-A-2 251 695**
**GB-A-1 259 503**
**GB-A-1 417 272**

**TETRAHEDRON, vol. 38, no. 11, 1982, Pergamon Press, OXFORD (GB), G.B. MARINI BETTOLO et al.: "Research on African Medicinal Plants", "hypoxoside, a new glycoside of uncommon structure from hypoxis obtusa busch", pages 1683-1687**

**S.N. WALFORD, Der Deutsche Apotheker, 31 (11), Nov. 79, p. 642-651**

(73) Proprietor: **Roecar Holdings (Netherlands Antilles) N.V.**
**Rokin 84**
**Amsterdam - C (NL)**

(72) Inventor: **Drewes, Siegfrid**
**76 Blackbarrow Road**
**Pietermaritzburg (ZA)**
Inventor: **Liebenberg, Roelof Wilke**
**16 Andre Street Pierneef Park**
**Johannesburg (ZA)**

(74) Representative: **Siewers, Gescha, Dr.**
**Rechtsanwälte Dr.Harmsen, Dr.Utescher Dipl.-Chem.Harmsen,Bartholatus Dr. Schaeffer,Dr.Fricke Patentanwälte Dr.Siewers,Dipl.Ing.Schöning Adenauerallee 28**
**D-2000 Hamburg 1 (DE)**

(56) References cited:
**M. BRÄUER et al., Fortschr. Med. 1978, 96 (15), p. 833-834**

**CANCER TREATMENT REPORTS, vol. 60, no. 8, August 1976, A.S. BARCLAY et al.: "Distribution of Anticancer Activity in Higher Plants", pages 1081-1113**

EP 0 092 226 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to pharmaceutical compositions for use in the treatment and prevention of cancerous conditions.

Extracts of plants of Hypoxidaceae family and especially Hypoxis rooperi have long been used in traditional African medicine for the treatment of prostata adenoma and other tumors as for example referred to in H. Bräuer et al., Fortsch. med. 1978, 96 (15), pages 833—834. Furthermore, extracts of plants of the Hypoxidaceae family have been found to be active in vivo screening tests with mouse P388 for the treatment of lymphocytic leukemia as described by A. Barclay et al., Cancer Treatment Reports 1976, 60, 1081—1113 "Distribution of Anticancer Activity in Higher Plants". For the purposes of this specification lymphocytic leukemia is not regarded as a cancerous condition, and if it is, such condition is excluded from the scope of this invention. Moreover, extracts of the Hypoxis genus have also been used for antiinflammatory and prostata hypertrophy treatment as for example referred to in U.K. patents 1259503, 1417272 or 2039217.

It has now been found that extracts of plants of the Hypoxidaceae family contain glycosides of an unsaturated compound corresponding to the following formula (I)

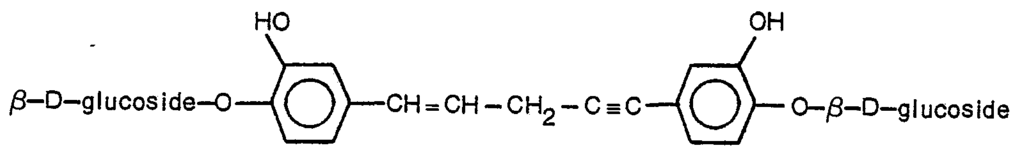

It is believed that all species of the Hypoxidaceae family are useful in this respect but particularly good results have been obtained using extracts from species of the Hypoxis genus such as Hypoxis acuminata, Hypoxis nitida, H. obtusa-nitida, H. latifolia, H. rigiduli, H. rooperi. In particular, an ethanol extract of H. rooperi and Spiloxene schlechteri gave excellent results.

The extraction of the corms of the plants can be carried out with water or an alcohol or a mixture thereof. After filtration, concentration in vacuo and freeze-drying the crude extraction material is separated by preparative HPLC using a reverse phase column. The crystalline diglucoside is characterized by its melting point of 147—148°C and optical rotation of $[\alpha]^{20}$ −110° (C=0,45 in methanol).

Dosages vary from individual to individual and also on the severity of the cancerous condition. However, it has been found that daily dosages from 600—2,400 mg of the powder obtain from extractions of plants or 300—1,200 mg of the diglucoside are effective.

The invention will now be described with reference to the following examples

Example 1

Freshly pulped corms of *Hypoxis rooperi* (10 kg) were extracted with ethanol (25 l) at room temperature for 48 hours. Filtration, and concentration *in vacuo*, followed by freeze-drying gave a brown powder (729 g). This crude material (10) was separated by preparative HPLC using a reverse-phase column (PrepPAK-500/$C_{18}$) with MeOH—$H_2O$ (50:50) as solvent and gave 6.9 g of an enriched fraction. Final separation took place by separating 10 g portions of this material on a Si gel 60 column (230—400 mesh 45 mm × 600 mm) with butan-2-ol-$C_6H_6$-$H_2O$-MeOH (4:3:2:1) as solvent. Typically a 10 g fraction yielded 7.9 g (40% from the fresh corms) of crystalline (I) from butan-2-ol, m.p. 147—148°C, $[\alpha]^{20}$ −110° (α, 0.45 in methanol). (Found: C. 54.18, H. 6.01, Calc for $C_{29}H_{34}O_{14}.2H_2O$: C. 54.20; H. 5.96; $H_2O$5.61%); $\nu_{max}$ (KBr): 3150—3500 (H bonded OH) $cm^{-1}$ λ max (MeOH): 247, 257 and 287 nm; δ ($CD_3OD$), 3.10—4.05 (14H; m, 2 protons on C-3 and 12 glucosylprotons), 4.75 (2H, m, 2 protons on C-1), 6.06 (H, dt, J=15.0 and J=5.0 Hz, proton on C-4), 6.53 (1H, d, J=15Hz, proton on C-5), 6.65—6.90 (4H, m, Arom H), 7.07 (2H, d, Arom H).

Example 2

The following further extractions were carried out for purposes of evaluation of the best methods and conditions of extraction.

a) Grated corms (10 kg) of *Hypoxis rooperi* were air dried and the product (2.95 kg) was extracted for 1 hour with boiling water (20 l). Spray drying of this extract provided a partially water soluble light brown powder (550 g), which contained 20% of Compound (I).

b) Gums obtained by centrifuging off the solid material from macerated corms (10 kg) of *H, rooperi* were diluted with cold water (1.5 l, 5°C) and the centrifugate (4 l) was spray dried to yield a partially water soluble light cream coloured powder (900 g) which contained 30% of Compound (I).

c) Pulped corms of *Hypoxis obtusa-nitida* (10 kg) were extracted with water (15 l, 25°C) with occasional stirring for 6 hours. The filtered extract (12.78 l) was freeze dried to yield a partially water soluble light brown powder (757 g) which contained 35% of Compound I. The residual wet pulp (10.8 kg) was extracted with boiling absolute ethanol (10.8 l) for 20 minutes. The filtered extract was concentrated under vacuum to remove most of the alcohol, diluted with water (1 litre), and then freeze dried to provide an almost completely water soluble light brown powder (361 g) which contained 43% of Compound (I).

d) Pulped corms of *H. obtusa-nitida* (10 kg) were extracted with boiling water (15 l) for 5 minutes. The filtrate (10.4 l) was freeze dried to provide a partially water soluble light brown powder (742 g) which contained 37% of Compound (I).

e) Pulped corms of *H. obtusa-nitida* (10 kg) were extracted with absolute ethanol (25 l, 25°C) for 66 hours with continous stirring. The filtrate (23 l) was concentrated under vacuum to remove most of the alcohol, diluted with water (1 litre) and then freeze dried to provide a water soluble dark brown powder *(i)* (630 g) which contained 45% of Compound (I). The residual moist pulp (8.05 kg) was extracted with boiling absolute ethanol (20 l). The filtrate treated as described above was freeze dried to provide an almost completely water soluble dark brown powder *(ii)* (99 g) which contained 42% of Compound (I).

f) Thinly spaced corms of *H. obtusa-obtusa* ((10 kg) were freeze dried to provide dried material (3.086 kg). This dried material was milled and a portion (300 g) was extracted with boiling 50% aqueous ethanol (5 l) for 5 minutes. The filtrate was treated as described above and freeze dried to provide a water soluble light cream powder (116 g) which contained 35% of Compound (I).

g) Pulped corms of young *H. obtusa-nitida* were extracted with boiling water (5 l) for 5 minutes. The filtrate on freeze drying provided a partially water soluble light brown powder (54 g) which contained 32% of Compound (I).

h) An extraction of young *H. obtusa-nitida* corms as described under (g) above, but prolonging the boiling process to 20 minutes, resulted in a relatively insoluble dark brown powder (82.3 g) which contained 27% of Compound (I).

i) Fresh *Hypoxis acuminata* corms are pulped directly into ethanol at room temperature (1.5 kg corms per 2 l ethanol). After stirring the pulp for 1 hour it is filtered. The filtrate is evaporated under vacuum (30 KPa) at elevated temperatures (70°—85°C). The obtained residue is freeze dried to provide a yellow-brown product in 7—10% yield from the fresh corms. The content of I on this extract varies between 42—57%. These yields depend on seasonal variations.

j) Fresh *Hypoxis acuminata* corms are sliced directly into liquid nitrogen. The frozen slices are freeze-dried and milled to a fine powder. This powder is then extracted with aqueous alcohol (1 part water to 2 parts of ethanol or methanol) using 6 l of the solution per 1 kg of plant powder. After 30 minutes stirring the mixture is filtered. Evaporation of the solution from the filtrate as described in Example 2i provides a yellowish-brown product in 9—12% from the original fresh corms. The content of I of this extract varies between 49—56%. Yields depend on seasonal variations.

k) Pulped corms of *Spiloxene schlechteri* on treatment with aqueous ethanol provided an extract which contained a substantial amount of Compound (I).

The above evaluations were obtained by comparative thin layer chromatography and HPLC analysis.

## Example 3

In order to demonstrate the activities of the extracts of the invention, cytotoxicity tests were carried out on M(52)B cell culture systems in vitro.

Mouse Sarkoma cells M(52)B were cultured as monolayers in M.E.M. with 106 foetal calf serum. After confluency was achieved (usually within 3—4 days depending on the number of cells added per bottle) the cells were trypsinized. Once loose the cell suspension was treated with M.E.M. containing 10% foetal calf serum in order to inactivate the trypsin.

An arbitrary volume of the suspension was then transferred to fresh nutrient solution rendering a final volume of 5 $cm^3$.

The test material was added at different concentrations to either the freshly prepared cell suspension or to the 24 hour developed cell culture in which the monolayer had a confluency of at least 40 to 50%.

All procedures were carried out under sterile conditions in a laminar flow chamber.

### Results

The addition of adequate amounts of the test material resulted in cell death within 24 hours. In other words the transferred cells failed to attach themselves on the bottom surface of the tissue culture flask, while the untreated control cells formed a confluent monolayer within 48—72 hours.

When less than adequate amounts of the test material were added some regeneration of the cells became apparent in the culture flasks over the first 24 hours. However, in these cases it took more than 10 days to achieve a 40—60% confluency, indicating that the larger proportion of the transferred cells had been completely destroyed.

TABLE 1
Percentage confluency due to H. obtusa-nitida (extracts ex example 2e(i)

| Agent | Dose mg/ml | Observation period in days confluency in % 1 | 3 | 6 | 10 | 14 days |
|---|---|---|---|---|---|---|
| Control | nil | 60 | 90 | split at 4 days | — | — |
| Extract from example 2e (i) | 0.4 | few growing cells | ± 5 | ± 10 | ± 40 | 30—80 |
| | 0.8 | (?) | n/g | n/g | n/g | + |
| | 1.6 | n/g | n/g | n/g | n/g | n/g |
| | 2.4 | n/g | n/g | n/g | n/g | n/g |

n/g No observed growth, with only isolated cells attached to the bottom surface of tissue culture flask.

The extracts of the invention may be administered as such but preferably in the form of their spraydried or freeze dried powders, in which case they may be provided in the form of tablets, capsules, dragees, creams, powders, ointments and any other usual form of medicament. The recognised additives may also be present to improve tabletting and capsuling. Dosage units may contain from approximately 100 mg to approximately 50 mg to extract per unit dose, and the rate of administration is 1 to 3 units, 3 times per day.

Example 4a

Acute toxicity evaluation of the Hypoxis extract

Toxicity tests were carried out with solutions prepared from extracts as described in example 2e(i) using groups of mice (equal numbers of males and females).

The weight of the animals ranged between 22 and 30 g. The follow-up observation period extended over 7 days following the administration of a single dose per animal.

The prepared solutions were filtered through Millex filter membranes (0.45 mm) before they were administered intravenously in order to remove any particles which may complicate the interpretation of the results, and to obtain a sterile solution.

For the oral or intraperitoneal route the solutions were administered as prepared, i.e. without prior filtration.

The results are presented in Table 2.

TABLE 2
Cumulative percentage mortality due to H. obtusa-nitida extract (example 2e(i)

| Dose mg/kg | Route of admin | Cumulative % mortality Days 1 | 2 | 3 | 5 | 7 |
|---|---|---|---|---|---|---|
| 500 | P.O | 0 | 0 | 0 | 0 | 0 |
| 1000 | P.O. | 0 | 0 | 0 | 0 | 0 |
| 2000 | P.O. | 0 | 0 | 0 | 0 | 0 |
| 500 | I.P. | 0 | 0 | 0 | 0 | 0 |
| 1000 | I.P. | 10 | 20 | 20 | 20 | 20 |
| 2000 | I.P. | 60 | 75 | 80 | 80 | 80 |
| 500 | I.V. | 0 | 0 | 0 | 0 | 0 |
| 1000 | I.V. | 20 | 25 | 25 | 25 | 25 |
| 2000 | I.V. | 80 | 100 | — | — | — |

Example 4b

Acute toxicity evaluation of (I)

Solutions of (I) in distilled water were prepared with the required concentration. Administered volumes were kept constant at the rate of 10 $cm^3$ per kg. In each case equal numbers of males and females were treated with a single dose. All animals were observed over 7 days for signs of toxic and/or pharmacodynamic effects.

A necropsy was performed on all surviving animals.

Results are presented in the Table.

TABLE 3

Acute toxicity evaluation of (I) on oral, intraperitoneal, and intravenous administration.

| Species | Administration Mode | Dose mg/kg | Mortality (Range %) | Remarks |
|---|---|---|---|---|
| Mouse | Oral | 1000<br>2000<br>4000 | 0/20<br>0/20<br>0/20 | No macroscopic lesions were found in any animal |
| Mouse | Intraperitoneal | 250<br>500<br>1000<br>2000 | 0/10<br>0/20<br>5/30 (10—30)<br>27/30 (80—100) | Following administration all animals exhibited signs of discomfort which increased in degrees with increasing doses |
| Rabbits | Intravenous | 50<br>100 | 0/2<br>0/2 | No visible effects were observed and no macroscopic lesions were found in these animals. No thrombophlebitis was found at the injection site. |

Conclusions:

It appears that (I) exhibits a low degree of direct toxic effects. No macroscopic lesions were observed no necropsy of the surviving animals.

Example 4c

Foetotoxic — Teratogenic effect of (I)

Two groups of six fully impregnated mice (Swiss Webster offspring) were treated orally with aqueous sôlutions of (I) once per day (10 ml/kg) starting day 1 through to day 8 of the gestation period. On day 20 of the pregnancies the mice were lightly anaethetised with diethyl ether and the uterus exposed by caesarian section. The total number of implantation sites were counted. All foeti were removed and examined for

(a) number alive and

(b) macroscopic gross anatomical abnormalities of limbs and head.

The results were compared to a control group and are presented in Table 4.

TABLE 4
Effects of orally administered (I) on pregnant mouse foeti

| GROUP | CONTROL | COMPOUND I | |
|---|---|---|---|
| | $H_2O$ only | 20 mg/kg | 100 mg/kg |
| No of animals | 6 | 6 | 6 |
| No of implantation sites | 56 | 58 | 58 |
| No of foeti | 52 | 52 | 49 |
| No of live foeti | 51 | 50 | 45 |
| No of malformations | 1 (?) | 0 | 3 (2?) |

Example 4(d)
"In vitro" cytostatic/cytotoxic effects with Hypoxis rooperi extracts (example 2(e)(i)) and Compound I

Four different cell culture types were used:

i. Mouse sarcoma — Ms 52B.
ii. Mouse melacoma — Mel B1—6.
iii. Heha 229.
iv. Human foetal foreskin fibroblasts — HFSF (non-malignant).

Procedure: Trypsinized cells were harvested into MEM and suitably diluted to supply about $2 \times 10^5$ cells per $cm^3$. A culture flask containing 4,5 $cm^3$ of culture medium was seeded with 0:5 $cm^3$ of the prepared cell suspension. This cell culture was then incubated at 37°C for 24 hours before the test substances dissolved in 0.5 $cm^3$ of the culture medium were added. All cultures were grown until an apparent 100% was achieved in the control flasks. In all cases presented in TABLE 5 cell growth was retarded considerably and growth regression was observed for MS 52 B. Mel B1—6 and Heha 229 cell cultures.

TABLE 5

| Incubation temp: 37°C | Confluency | | | | | | | |
|---|---|---|---|---|---|---|---|---|
| Cell culture type | MS 52 B | | Mel B1—6 | | Heha 229 | | HFSF | |
| test material | Ext. | I | Ext. | I | Ext. | I | Ext. | I |
| amounts added in ng | 80 | 40 | 80 | 40 | 80 | 40 | 100 | 40 |
| Hours: 24 | 50 | 40 | 40 | 40 | 50 | 50 | 40 | 40 |
| 48 | 40 | 20 | 40 | 30 | 50 | 60 | 60 | 50 |
| 72 | 20 | 20 | 20 | 40 | 40 | 50 | 75 | 50 |
| 96 | 20 | 20 | 5 | 40 | 20 | 30 | 60 | 40 |
| 120 | — | — | — | 30 | — | — | — | — |
| | In all cases controls had reached 100% confluency by 96 hours.<br>Ext. = *Hypoxis rooperi* extract as prepared in example 2e(i)<br>I = pure Compound I as described in example 1. | | | | | | | |

Despite the small number of animals tested it seems unlikely that at the doses administered (I) exhibits teratogenic effects. There were no indications of abnormally reduced growth rate of the foeti in any of the treated groups. No adverse effects were detected in the treated mother animals.

Example 5

Hypoxis extracts prepared as described in Example 2 were used to treat individual patients with various types of cancer, with the following surprising results.

a) A 64 year old male patient who had suffered from skin cancer (face and arms) had developed an inoperable growth near his anus and had been operated on for growths on his cheeks and lips. After treatment had commenced with an extract prepared as described in Example 2a (200 mg doses three times per day) his condition improved in that all cancerous growth started to regress within the first three weeks to finally disappear completely. The patient was treated six years ago and he is still alive and well.

b) The medical case history of a man, now aged 79, reads as follows:—

At age 70 the patient had a laparotomy and colostomy. Findings: a huge mass in the sigmoid colon, rectosigmoid junction and rectum. Due to extensive peritoneal infiltration of the tumor and the fact that obstruction was present it was decided to biopsy several of the excrescences and to perform proximal colostomy. The pathology report recorded the presence of lymphosarcoma and this was later confirmed by an X-ray investigation. Surgery was recommended to be followed by post-operative radiation treatment. The surgeon on performing an exploratory laparotomy found a huge mass within the sigmoid colon and upper rectum. It had infiltrated onto the posterior abdominal and lateral pelvic walls making a hard unresectible mass. After this operation the patient was not expected to live longer than two months. At this stage treatment commenced with a *Hypoxis rooperi* extract as described in Examples 2a and 2b with a daily dose of 3 times 200 mg. Four months later the patient was still alive and underwent surgery to free the colostomy from the abdominal wall and carry out a large bowel resection; no additional abnormalities were found in the rest of the peritoneal cavity. A pathology report on the excised 'intussuscepted' large bowel section recorded that the intussusception was oedematus and indurated (hardened) while the histology report records that sections of this bowel specimen showed congestion and superficial ulceration of the mucosa, but no evidence of lymphoma or malignancy. Since then the patient has continued to use Hypoxis extracts intermittently and he has regained his weight. He is now 79 years old and is active and healthy. There are no signs of a recurrence of the cancer.

c) An inoperable lung cancer was diagnosed in a middle aged female seven years ago. She commenced with a *Hypoxis rooperi* extract prepared as described in example 2a with a dose of 600 mg per day. She received no chemotherapy or radiation treatment. Within a month the patient reported a subjective improvement and within six months the patient had recovered completely. This patient is still alive, she takes the Hypoxis extract regularly and reports at least twice a year.

d) A bed-ridden male patient underwent radiation treatment for an inoperable lung cancer and he was not expected to survive for longer than two months. Treatment with *Hypoxis rooperi* extract prepared as described in Example 2a and 2b using 600 mg per day resulted in an improvement enabling the patient to leave his bed and walk around. He was still alive six months later when due to lack of communication, treatment with the extract was discontinued. Within two weeks the patient had to return to his bed. Treatment was resumed and within a week the patient was well enough to walk around again. Four months later this patient again discontinued treatment and he died shortly thereafter.

e) A 33 year old female patient after a radical mastectomy followed by radiation and chemotherapy developed metastatic cancer in the spine resulting in the destruction of one vertebra. Further chemotherapy was refused by the patient who commenced treatment with a *Hypoxis obtusa-nitida* extract prepared as described in Example 2e(i) at a dose of 3 × 300 mg per day.

A month later a bone scan was performed, no sign of the cancer could be found and the patient was operated on within a week to rebuild the vertebra with a hip-bone transplant. No sign of a residual cancer could be found during this operation.

f) Two females both suffering from severe skin cancer were treated with a *Hypoxis rooperi* extract prepared as described in Example 2a at a dose of 3 × 200 mg per day. Their condition cleared up within 60 days.

Example 6

Two female patients with histo-pathological confirmed carcinomas were treated with 600 mg Hypoxis extract 4 times per day. Both patients were considered incurable when the Hypoxis extract treatments commenced, and they then received no other cytostatic drugs or radiation treatment other than medication to relieve their general state of debilitation and pain. The Hypoxis extract contained 52% (I) and was prepared from *H. acumirata* as described in example 2e i.

a) A 77 year old patient had an infiltrating growth of a solid mammary carcinoma with pleural metastases and with distinct effusion. After 64 days treatment with the Hypoxis extract regression of the effusion was obvious together with a reduction of the metastases; there had been no need for drainage. The overall condition of the patient had improved and she reported to be completely free from pain.

b) A 54 year old patient had a serious papillary ovarian adeno-carcinoma accompanied by peritoneal and mesenterial infiltrations as well as ascites. After 2½ months treatment with the Hypoxis extract the general condition of the patient had improved. The aescites had regressed without the aid of drainage or diuretica.

**Claims for the Contracting States: BE CH DE FR GB IT LI LU NL SE**

1. Compound of the formula

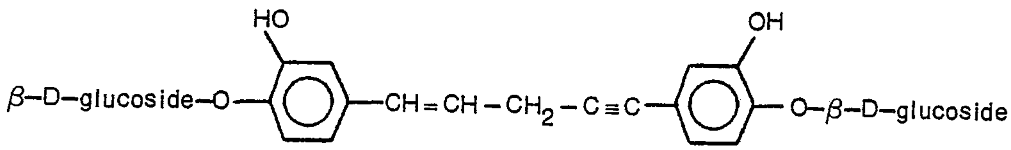

2. Use of a compound according to claim 1 for preparing medicaments for preventing or treating cancerous condition except leukemia.

**Claims for the Contracting State: AT**

1. Process for obtaining a compound of the following formula:

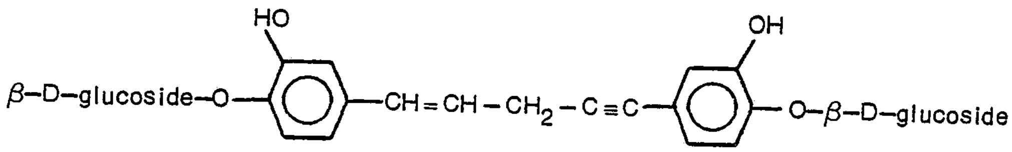

characterized in that the corms of plants of the family Hypoxidaceae are subjected to an extraction process using water, alcohol or aqueous alcohol.

2. Process according to claim 1, characterized in that the plants are of the genus Hypoxis nitida, Hypoxis obtusa-nitida, Hypoxis acuminata, Hypoxis latifolia, Hypoxis regiludi, Hypoxis rooperi, Hypoxis obtusa-obtusa or Spiloxene schlechteri.

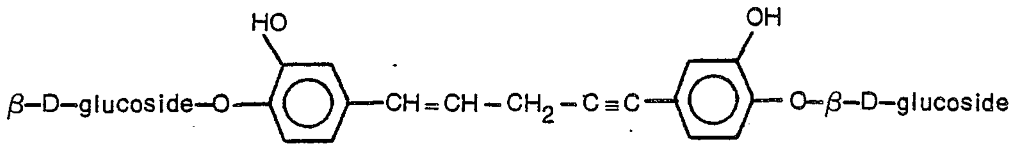

**Patentansprüche für die Vertragsstaater: BE CH DE FR GB IT LI LU NL SE**

1. Verbindung gemäß nachstehender Formel

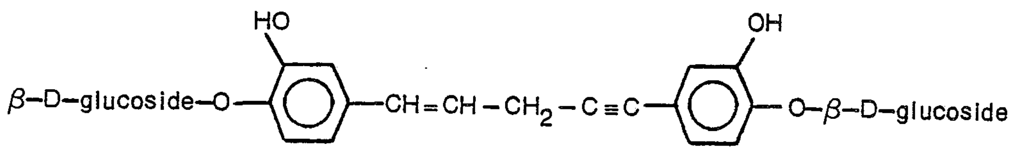

2. Verfahren nach Anspruch 1 zur Herstellung von Medikamenten zur Verhütung oder Behandlung von Krebs, außer Leukämie.

**Patentansprüche für den Vertragsstaaten: AT**

1. Verfahren zur Gewinnung einer Verbindung der nachfolgenden Formel:

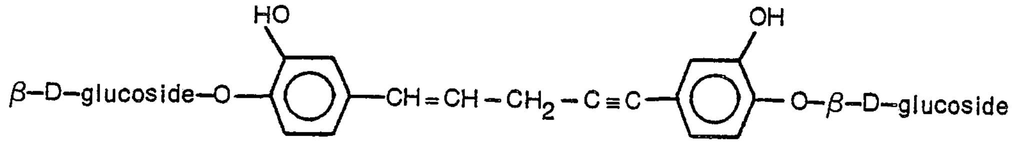

dadurch gekennzeichnet, daß man Cormi von Pflanzen der Familie Hypoxidaceae am Extraktionsprozess unter Verwendung von Wasser, Alkohol oder wässrigem Alkohol unterwirft.

2. Verfahren nach Anspruch 1, dadurch gekennzeichnet, daß man als Pflanze Hypoxis nitida, Hypoxis obtusa-nitida, Hypoxis acuminata, Hypoxis latifolia, Hypoxis regiludi, Hypoxis rooperi, Hypoxis obtusa-obtusa oder Spiloxene schlechteri einsetzt.

**Revendications pour les Etats contractant: BE CH DE FR GB IT LI LU NL SE**

1. Composé de la formule suivante

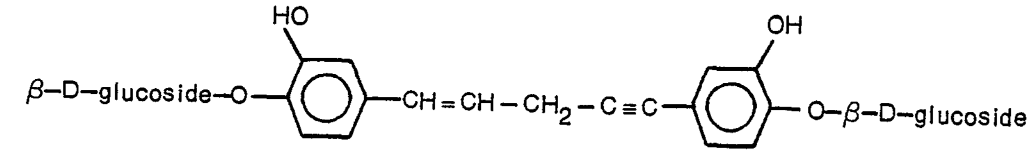

2. Utilisation d'un composé selon la revendication 1 pour la préparation des médicaments pour la prévention et le traitement des maladies cancéreuses à l'exception de la leucémie.

**Revendications pour l'Etat contractant: AT**

1. Procédé pour préparer un composé de la formule suivante:

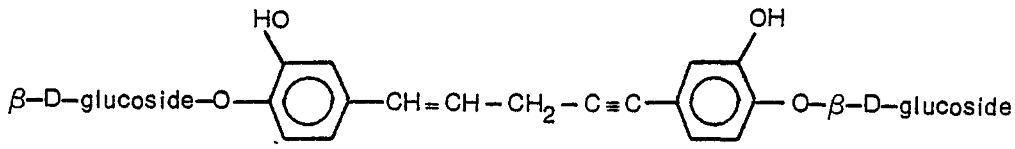

caracterisé par la soumission des cormes des plantes de la famille Hypoxidaceae à un processus d'extraction en utilisant de l'eau, de l'alcool ou de l'alcool aqueux.

2. Procédé selon la revendication 1, caractérisé par l'utilisation les plantes Hypoxis nitida, Hypoxis obtuda-nitida, Hypoxis acuminata, Hypoxis latifolia, Hypoxis regiludi, Hypoxis roouperi, Hypoxis obtusa-obtusa ou Spiloxene schlechteri.